# EUROPEAN PATENT APPLICATION

(11) **EP 4 006 151 A1**
(43) Date of publication of application: **01.06.2022**
(21) Application number: 20846089.9
(22) Date of filing: 18.06.2020
(51) Int. Cl.: C12N 15/09, C12Q 1/686, C12Q 1/6886, G01N 33/574

(54) **ESOPHAGEAL CANCER BIOMARKER AND USE THEREFOR**

(30) Priority: 29.07.2019 JP 2019139247
(71) Applicant: Public University Corporation Nagoya City University, Mizuho-ku Nagoya-shi Aichi 467-8601 (JP)
(72) Inventor: SHIMURA, Takaya, Nagoya-shi, Aichi 467-8601 (JP); OKUDA, Yusuke, Nagoya-shi, Aichi 467-8601 (JP); IWASAKI, Hiroyasu, Nagoya-shi, Aichi 467-8601 (JP); KATAOKA, Hiromi, Nagoya-shi, Aichi 467-8601 (JP)
(74) Representative: Papula Oy
(86) International application number: PCT/JP2020/024027
(87) International publication number: WO 2021/019944

(57) **Abstract**

A highly practical biomarker that is specific to esophageal cancer and minimally invasive is provided. hsa-miR-619-5p in urine, hsa-miR-1273f in urine, hsa-miR-3135b in urine, hsa-miR-4327 in urine, and hsa-miR-150-3p in urine were identified as esophageal cancer biomarkers. Esophageal cancer contraction is determined by using the expression level of these microRNAs as an index.

SEQUENCE LISTING

<110>  NAGOYA CITY UNIVERSITY

<120>  BIOMARKER FOR ESOPHAGUS CANCER AND USE THEREOF

<130>  NC19001P

<150>  JP P2019-139247
<151>  2019-07-29

<160>  7

<170>  PatentIn version 3.5

<210>  1
<211>  22
<212>  RNA
<213>  Homo sapiens

<400>  1
gcugggauua caggcaugag cc                                                22


<210>  2
<211>  19
<212>  RNA
<213>  Homo sapiens

<400>  2
ggagauggag guugcagug                                                    19


<210>  3
<211>  22
<212>  RNA
<213>  Homo sapiens

<400>  3
ggcuggagcg agugcagugg ug                                                22


<210>  4
<211>  19
<212>  RNA
<213>  Homo sapiens

<400>  4
ggcuugcaug ggggacugg                                                    19


<210>  5
<211>  22
<212>  RNA
<213>  Homo sapiens

<400>  5
cugguacagg ccugggggac ag                                                22


<210>  6
<211>  22
<212>  RNA

<213>  Homo sapiens

<400>  6
uguguccggg aaguggagga gg                                                22

<210>  7
<211>  23
<212>  RNA
<213>  Homo sapiens

<400>  7
agggugggc uggaggugg gcu                                                 23

## Description

### [Technical Field]

The present invention relates to a useful indicator for examination and diagnosis of esophageal cancer and use thereof. More particularly, the present invention relates to an esophageal cancer biomarker and an examination method that utilizes the biomarker. Priority is claimed on Japanese Patent Application No. 2019-139247, filed July 29, 2019, the content of which is incorporated herein by reference.

### [Background Art]

The gold standard for the diagnosis of esophageal cancer is upper gastrointestinal endoscopy and pathological diagnosis using biopsy tissue; however, since symptoms do not appear when esophageal cancer does not progress and cause passage obstruction, the cancer is found to be in a highly progressed state when examined by endoscopy at the time of manifesting symptoms, so that the risk of metastasis is high. Therefore, esophageal cancer is one of the diseases with poor prognosis. During upper gastrointestinal contrast radiography performed for gastric cancer examination, only one or two photographs of the esophagus are usually taken, and it is difficult to detect esophageal cancer unless a tumor is discovered in the advanced stage that causes passage obstruction.

Serum markers such as SCC, CYFRA21-1, and anti-p53 antibodies are used in blood tests; however, these serum markers are not considered to have sufficient sensitivity and specificity. Furthermore, although the development of biomarkers specific to esophageal cancer is underway (for example, Patent Documents 1 to 3), there are no examples of clinical application. On the other hand, it has been reported that a combination of specific microRNAs in serum is useful for diagnosing esophageal cancer (Non-Patent Document 1).

### [Citation List]

### [Patent Documents]

[Patent Document 1]
   Pamphlet of PCT International Publication No. WO 2015/194627
[Patent Document 2]
   Pamphlet of PCT International Publication No. WO 2015/098112
[Patent Document 3]
   Japanese Unexamined Patent Application, First Publication No. 2018-123136

### [Non-Patent Document]

[Non-Patent Document 1]
JAMA Netw Open. 2019 May 3; 2(5):e194573. doi:10.1001/jamanetworkopen.2019.4573.

### [Summary of Invention]

### [Technical Problem]

As described above, in connection with esophageal cancer in the current situation, there is no biomarker which can be easily and routinely used in clinical settings and medical examinations. Therefore, it is an object of the present invention to provide a highly practical biomarker that is specific to esophageal cancer and is minimally invasive.

### [Solution to Problem]

The inventors of the present invention have proceeded with studies aiming at creating a biomarker that is specific to esophageal cancer, while placing importance on enabling minimally invasive and simple tests. Specifically, the present inventors attempted to identify a biomarker specific to esophageal cancer from urine. Detailed and comprehensive analysis was carried out using more than 350 specimens, and as a result, it was found that specific microRNAs enable the determination of esophageal cancer with high specificity and sensitivity. That is, the present inventors succeeded in identifying biomarkers with excellent diagnostic ability for esophageal cancer. As an important point, it was suggested that each of the identified microRNAs be independently useful for the diagnosis of esophageal cancer. On the other hand, as a result of conducting further investigation, it was found that the identified microRNAs, except for one, are useful for the detection and diagnosis of early-stage (stage I) esophageal cancer. As a result of further investigation, it was demonstrated that among the five microRNAs identified, four (hsa-miR-619-5p in urine, hsa-miR-1273f in urine, hsa-miR-4327 in urine, and hsa-miR-150-3p in urine) are highly useful for the detection and diagnosis of early-stage esophageal cancer, and at the same time, there was suggested a possibility that the remaining one (hsa-miR-3135b in urine) can also be utilized for the detection and diagnosis of early-stage esophageal cancer. Based on the above-described results and consideration, the following inventions are provided.
[1] An esophageal cancer biomarker, comprising:
   at least one of microRNA selected from a group consisting of hsa-miR-619-5p in urine, hsa-miR-1273f in urine, hsa-miR-3135b in urine, hsa-miR-4327 in urine, and hsa-miR-150-3p in urine.
[2] A method for examining esophageal cancer comprising:
   using an expression level of the esophageal cancer biomarker according to [1] as an index.
[3] The esophageal cancer biomarker according to [1], in which the esophageal cancer biomarker is used for detecting early-stage esophageal cancer.
[4] The esophageal cancer biomarker according to [3], in which the esophageal cancer biomarker comprises hsa-miR-619-5p in urine, hsa-miR-1273f in urine, hsa-miR-4327 in urine, or hsa-miR-150-3p in urine.
[5] A method for examining early-stage esophageal cancer, comprising:
   using an expression level of the esophageal cancer biomarker according to [3] or [4] as an index.
[6] The method according to [2], further comprising:
   a step (1) which is a step of detecting the esophageal cancer biomarker in a specimen collected from an examinee and determining the expression level; and
   a step (2) which is a step of determining esophageal cancer contraction, based on the expression level determined in the step (1).
[7] The method according to [5], further comprising:
   a step (1) which is a step of detecting the esophageal cancer biomarker in a specimen collected from an examinee and determining the expression level; and
   a step (2) which is a step of determining early-stage esophageal cancer contraction, based on the expression level determined in the step (1).
[8] The method according to [6] or [7], in which in the step (1), the expression level of the esophageal cancer biomarker is determined by conducting standardization using hsa-miR-4669 and hsa-miR-6756-5p as standardization factors.
[9] The method according to any one of [6] to [8], in which the esophageal cancer biomarker to be detected in the step (1) is hsa-miR-619-5p in urine or hsa-miR-1273f in urine.
[10] The method according to any one of [6] to [9], in which the esophageal cancer biomarker is detected by a qRT-PCR method.
[11] A kit for esophageal cancer examination, the kit including:
   a reagent for detecting the esophageal cancer biomarker according to [1]; and
   an instruction manual.
[12] A kit for esophageal cancer examination, the kit including:
   a reagent for detecting the esophageal cancer biomarker according to [3] or [4]; and
   an instruction manual.
[13] The kit for esophageal cancer examination according to [11] or [12], in which the reagent is at least one selected from a group consisting of a reagent for detecting hsa-miR-619-5p in urine, a reagent for detecting hsa-miR-1273f in urine, a reagent for detecting hsa-miR-3135b in urine, and a reagent for detecting hsa-miR-4327 in urine, and/or a reagent for detecting hsa-miR-150-3p in urine.
[14] The kit for esophageal cancer detection according to any one of [11] to [13], the kit further comprising at least one of elements selected from a group consisting of a reagent for microRNA extraction, a standardization factor-specific primer, a DNA polymerase, a reverse transcriptase, dNTP, a urine specimen collecting vessel, a reaction device, and a detection device.

### [Brief Description of Drawings]

Fig. 1 shows a research and analysis scheme.
Fig. 2 shows the determination of esophageal cancer by means of miRNAs in urine.
Fig. 3 shows an ROC curve (diagnosis panel for miRNAs in urine). Healthy persons vs. esophageal cancer cases
Fig. 4 shows the determination of early-stage (stage I) esophageal cancer by means of miRNAs in urine.
Fig. 5 shows an ROC curve (diagnosis panel for miRNAs in urine). Healthy persons vs. stage I esophageal cancer cases.
Fig. 6 shows the correlation between the expression of miRNAs in urine and the clinical stage. Stage I: 17 cases, Stage II/III: 19 cases, Stage IV: 12 cases.
Fig. 7 shows the miRNA expression in culture fluids of normal esophageal cells and cancer cells. Het-1A: Human normal esophageal squamous cell line, TE-1: Human esophageal squamous carcinoma cell line, T.T: Human esophageal squamous carcinoma cell line, KYSE-30: Human esophageal squamous carcinoma cell line. (n = 8 to 11)
Fig. 8 shows the miRNA expression in tumor tissue and adjacent normal tissue. (N = 27)

### [Description of Embodiments]

### 1. Esophageal cancer biomarker

A first aspect of the present invention relates to an esophageal cancer biomarker. The term "esophageal cancer biomarker" as used herein refers to a biomolecule that serves as an indicator for esophageal cancer contraction. The biomarker of the present invention is useful for the detection of esophageal cancer (grasping of whether esophageal cancer is contracted), particularly for the detection of early-stage esophageal cancer.

The esophagus is a tubular organ that connects the pharynx and the stomach, and is roughly divided into cervical esophagus, thoracic esophagus, and abdominal esophagus. The wall of the esophagus is composed of mucosa (mucosal epithelium, lamina propria mucosae, and lamina muscularis mucosae), submucosa, muscularis propria, and adventitia. In the case of Japanese people, esophageal cancer is often found near the center of the esophagus. Esophageal cancer has a feature of easily undergoing lymph node metastasis, and when lymph node metastasis has occurred, metastasis is often found over a wide range. Esophageal cancer is subdivided according to the histological type, stage, and the like. A majority of incipient esophageal cancer cases have no subjective symptoms, and it is difficult to detect esophageal cancer at an early stage. For the diagnosis of esophageal cancer, a blood examination, an esophagographic examination, endoscopy, computed tomography (CT), MRI scan, PET scan, an ultrasonic examination, and the like are conducted.

The term "biomolecule" as used herein refers to a molecule (compound) found in a living body. In the present invention, a microRNA, which is a biomolecule, is used as a biomarker; however, on the occasion of utilizing the microRNA (typically applied to an examination method), this molecule in a specimen (sample) that has been separated from a living body is to be used. Urine is used as a specimen. Therefore, specific microRNAs in urine serve as biomarkers of the present invention.

A microRNA is a small molecular weight RNA of about 22 bases existing in a living body. A microRNA suppresses protein production by binding to a target gene having a partially complementary sequence in the 3' untranslated region (3'UTR) and suppressing mRNA destabilization and translation. The function of this microRNA constitutes a portion of an important mechanism for post-transcriptional expression regulation of genes.

The biomarker of the present invention is composed of a specific microRNA, namely, hsa-miR-619-5p, hsa-miR-1273f, hsa-miR-3135b, hsa-miR-4327, or hsa-miR-150-3p. Among them, hsa-miR-619-5p and hsa-miR-1273f are highly useful from the viewpoint of bringing about higher diagnostic accuracy.

The biomarker of the present invention is also useful for the detection of early-stage esophageal cancer, and particularly, the usefulness of hsa-miR-619-5p, hsa-miR-1273f, hsa-miR-4327, and hsa-miR-150-3p is high. Furthermore, even in this case, hsa-miR-619-5p and hsa-miR-1273f are highly useful. The disease stage of esophageal cancer is broadly divided into stages I, II, III, and IV. Unless particularly stated otherwise, the term "early-stage esophageal cancer" according to the present invention is stage I (I stage) esophageal cancer.

hsa-miR-619-5p, hsa-miR-1273f, hsa-miR-3135b, hsa-miR-4327, and hsa-miR-150-3p are each a microRNA having the following sequence.
hsa-miR-619-5p: GCUGGGAUUACAGGCAUGAGCC (SEQ ID NO:1)
hsa-miR-1273f: GGAGAUGGAGGUUGCAGUG (SEQ ID NO:2)
hsa-miR-3135b: GGCUGGAGCGAGUGCAGUGGUG (SEQ ID NO:3)
hsa-miR-4327: GGCUUGCAUGGGGGACUGG (SEQ ID NO:4)
hsa-miR-150-3p: CUGGUACAGGCCUGGGGGACAG (SEQ ID NO:5)

For the convenience of description, "hsa-" in the names of microRNAs may be omitted in the present specification.

### 2. Examination utilizing esophageal cancer biomarker

A second aspect of the present invention provides a method for examining esophageal cancer (hereinafter, also referred to as "examination method of the present invention"), in relation to the use application of the biomarker of the present invention. The examination method of the present invention is useful as means for conveniently detecting esophageal cancer. According to the examination method of the present invention, objective bases that enable determination of whether or not esophageal cancer is contracted (presence or absence of disease contraction) are provided. Furthermore, an aspect of the examination method of the present invention is useful for detecting early-stage esophageal cancer, and in the case of this aspect, objective bases useful for determining whether or not early-stage esophageal cancer is contracted are obtained. The information (examination results) provided by the examination method of the present invention is based on an objective indicator called a biomarker (biomolecule) that reflects the condition or change in the living body, and the information itself enables determination of esophageal cancer; however, it is also acceptable to conduct the final determination (typically a definitive diagnosis) by subsidiarily utilizing the information and also considering other indicators as necessary. In addition, in order to make a clearer distinction from a diagnosis including a doctor's determination, the examination method of the present invention can also be referred to as "a method assisting an esophageal cancer examination". The present invention provides information beneficial for the diagnosis of esophageal cancer by utilizing an objective (index) independently of a doctor's determination.

In the examination method of the present invention, the expression level of the biomarker of the present invention in a specimen derived from an examinee is used as an index. The term "level" as used herein typically means "amount" or "concentration". However, according to conventional practice and common technical knowledge, the term "level" is also used even in the case of indicating whether or not the molecule as an object of detection can be detected (that is, the presence or absence of apparent existence).

Typically, in the examination method of the present invention, the following steps (1) and (2) are carried out:
(1) a step of detecting the esophageal cancer biomarker in a specimen collected from an examinee and determining the expression level; and
(2) a step of determining esophageal cancer contraction based on the expression level determined in step (1).

### Step (1)

In step (1), a specimen collected from an examinee is prepared, the biomarker of the present invention (esophageal cancer biomarker) is detected, and the expression level is determined. As the biomarker, hsa-miR-619-5p, hsa-miR-1273f, hsa-miR-3135b, hsa-miR-4327, or hsa-miR-150-3p is used; however, in the case of aiming at the detection of early-stage esophageal cancer, it is preferable to employ hsa-miR-619-5p, hsa-miR-1273f, hsa-miR-4327, or hsa-miR-150-3p. Furthermore, in a particularly preferred embodiment, hsa-miR-619-5p or hsa-miR-1273f is employed. On the other hand, the examinee is not particularly limited. That is, the present invention can be widely applied to those for whom the presence or absence of esophageal cancer contraction needs to be determined. For example, those who have the possibility of contracting or are suspected of having contracted esophageal cancer, such as those who have signs of esophageal cancer or symptoms characteristic of esophageal cancer, as well as those who have no subjective symptoms (including healthy persons) may also be identified as examinees.

A specimen is collected prior to the implementation of the present invention. Urine is used as the specimen. The specimen may be collected and prepared by a conventional method.

In this step, a biomarker in the specimen is detected; however, it is not essential to strictly quantify the expression level of the biomarker. That is, it is sufficient when the biomarker can be detected to the extent that esophageal cancer contraction can be determined in the subsequent step (2). For example, detection can be carried out such that it can be determined whether or not the level of the biomarker in the specimen exceeds a predetermined reference value.

The method for detecting the biomarker is not particularly limited. For example, the biomarker can be detected by a method of utilizing a nucleic acid amplification reaction, which is represented by a qRT-PCR method, and a microarray. Regarding the nucleic acid amplification reaction, a Polymerase Chain Reaction (PCR) method or modifications thereof, as well as a Loop-Mediated Isothermal Amplification (LAMP) method (Tsugunori Notomi et al. Nucleic Acids Research, Vol.28, No. 12, e63, 2000; Kentaro Nagamine, Keiko Watanabe et al. Clinical Chemistry, Vol.47, No.9, 1742-1743, 2001), an Isothermal and Chimeric primer-initiated Amplification of Nucleic acids (ICAN) method (Japanese Patent No. 3433929, Japanese Patent No. 3883476), a Nucleic Acid Sequence-Based Amplification (NASBA) method, a Ligase Chain Reaction (LCR) method, a Self-sustained Sequence Replication (3SR) method, a Standard Displacement Amplification (SDA) method, a Transcription-Mediated Amplification (TMA) method, Rolling Circle Amplification (RCA), and the like can be employed.

The expression level of the biomarker is determined from the detection results. When determining the expression level, standardization is usually carried out in order to increase accuracy, objectivity, and the like. For standardization, the detection results for microRNAs which are recognized to undergo constant expression may be used. Preferably, hsa-miR-4669 and hsa-miR-6756-5p are used as standardization factors. That is, the detection result for the biomarker is corrected with the detection results for hsa-miR-4669 and hsa-miR-6756-5p, and the corrected result is used as the expression level to be used for the determination in step (2). In addition, RNU6B, which has been generalized as a standardization factor for tissues and cell lines, may also be utilized as a standardization factor.

In a case where the biomarker is measured by a qRT-PCR method, the Threshold Cycle (Ct) value may be utilized to determine the expression level. In this case, for example, a value (ΔCt value) obtained by subtracting the Ct value of the standardization factor (standardization factor Ct value) from the Ct value of the biomarker is determined, and the ΔCt value is used as the expression level to be used for the determination in step (2).

### Step (2)

In step (2), the esophageal cancer contraction is determined based on the expression level determined in step (1). In a case where the aim is to detect early-stage esophageal cancer, the contraction of early-stage esophageal cancer will be determined. In order to enable accurate determination, for example, the determination may be made while comparing the expression level obtained in step (1) with the expression level of a control (control specimen) or in comparison with the criteria set based on the expression level of the control. Regarding the control, for example, the biomarker expression level of a healthy person and/or the biomarker expression level of an esophageal cancer patient, or the biomarker expression level of an early-stage (stage I) esophageal cancer patient (in the case of aiming at the detection of early-stage esophageal cancer) can be used.

As shown in the Examples that will be described later, in all of hsa-miR-619-5p, hsa-miR-1273f, hsa-miR-3135b, hsa-miR-4327, and hsa-miR-150-3p, which serve as biomarkers of the present invention, a significant increase in expression was observed in the cases of esophageal cancer. That is, the expression level of the biomarker of the present invention shows a positive correlation with esophageal cancer contraction. Therefore, regarding the expression level of a biomarker, a high expression level serves as a basic index (determination criterion) for esophageal cancer contraction. On the other hand, hsa-miR-619-5p, hsa-miR-1273f, hsa-miR-4327 and hsa-miR-150-3p showed a significant increase in the expression in the cases of early-stage (stage I) esophageal cancer. Furthermore, also for hsa-miR-3135b, a significant increase in the expression was recognized in the cases of early-stage esophageal cancer as compared with healthy persons. Accordingly, the high expression level of these biomarkers is an index (determination criterion) for the contraction of early-stage esophageal cancer.

The determination in step (2) may be qualitative, semi-quantitative, or quantitative. Examples of qualitative determination and quantitative determination are shown below. The following example is an example of the case of determining esophageal cancer contraction; however, the same applies to the determination of early-stage esophageal cancer contraction. The number of determination categories, the expression level of the biomarker associated with each determination category, the determination results, and the like can be discretionally set through preliminary experiments and the like, without being bound by the following examples. Furthermore, the reference value and the cutoff value to be used for determination may be set while considering, for example, the specimen to be used, required accuracy (reliability), and the like. When setting the reference value or the cutoff value, a statistical analysis using a large number of specimens may be utilized. As is clear from the determination criteria, the determination here can be made automatically or mechanically without the determination by a person having specialized knowledge, such as a doctor or a laboratory technician.

### (Example of qualitative determination)

When the biomarker expression level is higher than the reference value A, it is determined that "esophageal cancer has been contracted", and when the biomarker expression level is equal to or lower than the reference value A, it is determined that "esophageal cancer is not contracted".

### (Example of quantitative determination)

As shown below, the chance of contraction (%) is set in advance for each range of the expression level of the biomarker, and the chance of contraction (%) is determined.
a < Expression level: The chance of esophageal cancer contraction is greater than 80%.
b < Expression level ≤ a: The chance of esophageal cancer contraction is 20% to 80%.

Expression level < b: The chance of esophageal cancer contraction is less than 20%.

As described above, when the qRT-PCR method is utilized for the detection of the biomarker in step (1), preferably, the determination of step (2) is carried out by using the value (ΔCt value) obtained by subtracting the Ct value of the standardization factor from the Ct value of the biomarker, as the biomarker expression level. For example, when the 2^{-ΔCt} value is equal to or higher than the reference value (cutoff value), it is determined that "esophageal cancer has been contracted" or "there is a high possibility of esophageal cancer contraction", and when the 2^{-ΔCt} value is lower than the reference value, it is determined that "esophageal cancer is not contracted" or "there is a low possibility of esophageal cancer contraction". The reference value as used herein (the numerical value of the 2^{-ΔCt} value that serves as the boundary of the high and low of the possibility of esophageal cancer contraction) is set for each of the microRNAs to be used as the biomarker. As described above, the reference value may be determined by utilizing a statistical analysis using a large number of specimens.

The determination results obtained in step (2) serve as useful information for the diagnosis of esophageal cancer and are helpful not only for the diagnosis of esophageal cancer but also for early detection of esophageal cancer and the determination of more appropriate treatment policy (selection of effective treatment method, and the like). The results may also provide an opportunity to undergo a detailed examination (for example, a secondary examination).

### 3. Kit for esophageal cancer examination

The present invention also provides a kit for esophageal cancer examination. The kit of the present invention includes a reagent for detecting the biomarker of the present invention (reagent for biomarker detection) as an essential element. An example of the reagent for biomarker detection is a biomarker-specific primer.

Preferably, reagents for detecting hsa-miR-4669 and hsa-miR-6756-5p as standardization factors, that is, a reagent for detecting hsa-miR-4669 (for example, a hsa-miR-4669-specific primer) and a reagent for detecting hsa-miR-6756-5p (for example, a hsa-miR-6756-5p-specific primer), are also preferably included in the kit.

An instruction manual is usually provided with the kit of the present invention. Other reagents (a reagent for microRNA extraction, a DNA polymerase, a reverse transcriptase, dNTP, and the like), devices or instruments (a urine specimen collecting vessel, a reaction device, a detection device, and the like), which are used at the time of performing a method for examining esophageal cancer, may also be included in the kit.

### [Examples]

In order to find biomarkers specific to esophageal cancer (esophageal cancer biomarkers), the following investigation was conducted.

### 1. Method

### (1) Target/method/clinical specimen

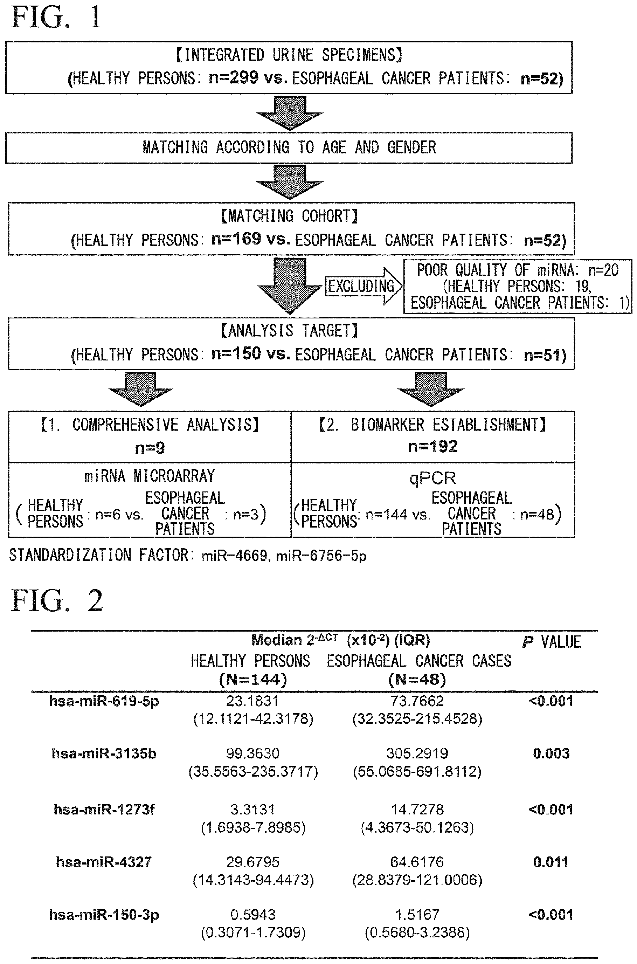

First, from a total cohort of 351 cases (healthy persons: 299 cases, esophageal cancer patients: 52 cases), 221 cases that matched for age and gender randomly at about 3: 1 were extracted, and 201 cases (healthy persons: 150 cases, esophageal cancer patients: 51 cases) obtained by excluding 20 cases for whom the quality of miRNA was poor and difficult to analyze, were designated as analysis targets. Those cases were randomly classified into two groups, namely, (i) a comprehensive analysis cohort of 9 cases (healthy persons: 6 cases, esophageal cancer patients: 3 cases), and (ii) a biomarker establishment cohort of 192 cases (healthy persons: 144 cases, esophageal cancer patients: 48 cases) (Fig. 1).

First, a miRNA array analysis was performed using the (i) comprehensive analysis cohort of 9 cases, and a plurality of miRNAs abnormally expressed in the urine of esophageal cancer cases were identified. Next, for the (ii) biomarker establishment cohort of 192 cases, each miRNA extracted in (i) was measured by a qPCR method, and meaningful esophageal cancer biomarkers were identified.

### (2) RNA extraction and cDNA synthesis

A miRNA was extracted from 200 µl of a urine specimen cryopreserved at - 80°C immediately after collection, by using a miRNeasy Serum/Plasma Kit (Qiagen, Valencia, CA) according to the protocol. Using the extracted miRNA as a template, cDNA synthesis was carried out by using a TaqMan Advanced MicroRNA cDNA Synthesis Kit (ThermoFisher Scientific, Waltham, USA) according to the protocol.

### (3) Microarray analysis

Analysis was conducted with a SurePrint miRNA microarray (Agilent, Santa Clara, USA), by using the extracted miRNA. GeneSpringGX (Agilent) was used for data analysis.

### (4) qPCR

qRT-PCR was performed using a 7500 Fast real-time PCR system (ThermoFisher Scientific, Watham, USA), by using the synthesized cDNA and using a TaqMan Advanced MicroRNA Assay. The thermal cycle and the sequences of the primers used are described in the following tables (Tables 1 and 2).

Thermal cycle of qRT-PCR

**[Table 1]**

| Step | Temperature | Time | Number of cycles |
|---|---|---|---|
| Enzyme activation | 95°C | 20 seconds | 1 |
| Modification | 95°C | 3 seconds | 40 |
| Annealing/extension | 60°C | 30 seconds | |

TaqMan Advanced MicroRNA Assays used

**[Table 2]**

| Assay name | Assay ID | Mature body miRNA sequence |
|---|---|---|
| hsa-miR-4669 | 478925_mir | UGUGUCCGGGAAGUGGAGGAGG (SEQ ID NO:6) |
| hsa-miR-6756-5p | 480284_mir | AGGGUGGGGCUGGAGGUGGGGCU (SEQ ID NO:7) |
| hsa-miR-619-5p | 479103_mir | GCUGGGAUUACAGGCAUGAGCC (SEQ ID NO:1) |
| hsa-miR-3135b | 478011 _mir | GGCUGGAGCGAGUGCAGUGGUG (SEQ ID NO:3) |
| hsa-miR-1273f | 478679_mir | GGAGAUGGAGGUUGCAGUG (SEQ ID NO:2) |
| hsa-miR-4327 | 478894_mir | GGCUUGCAUGGGGGACUGG (SEQ ID NO:4) |
| hsa-miR-150-3p | 478721 mir | CUGGUACAGGCCUGGGGGACAG (SEQ ID NO:5) |

### (5) Standardization factors

As the standardization factors, it was decided to use miR-4669 (SEQ ID NO:6) and miR-6756-5p (SEQ ID NO:7) detected by a global normalization method of the miRNA expression signal in urine obtained with a miRNA microarray. During the analysis, the Ct values of miR-619-5p, miR-1273f, miR-3135b, miR-4327, and miR-150-3p were measured by qRT-PCR, and the ΔCt values were calculated by subtracting the standardized Ct value (average Ct value of miR-4669 and miR-6756-5p) from each of the Ct values. The expression difference from the standardized Ct value: 2^{-ΔCt} (because the ΔCt value can be converted to the expression level of each of the miRNAs in each specimen) is used as the expression level of each miRNA, and esophageal cancer is detected and diagnosed according to the magnitude of this expression level. Since miR-4669 and miR-6756-5p are constantly and stably expressed in urine and serum, they are useful as standardization factors for miRNA in body fluids.

### 2. Results

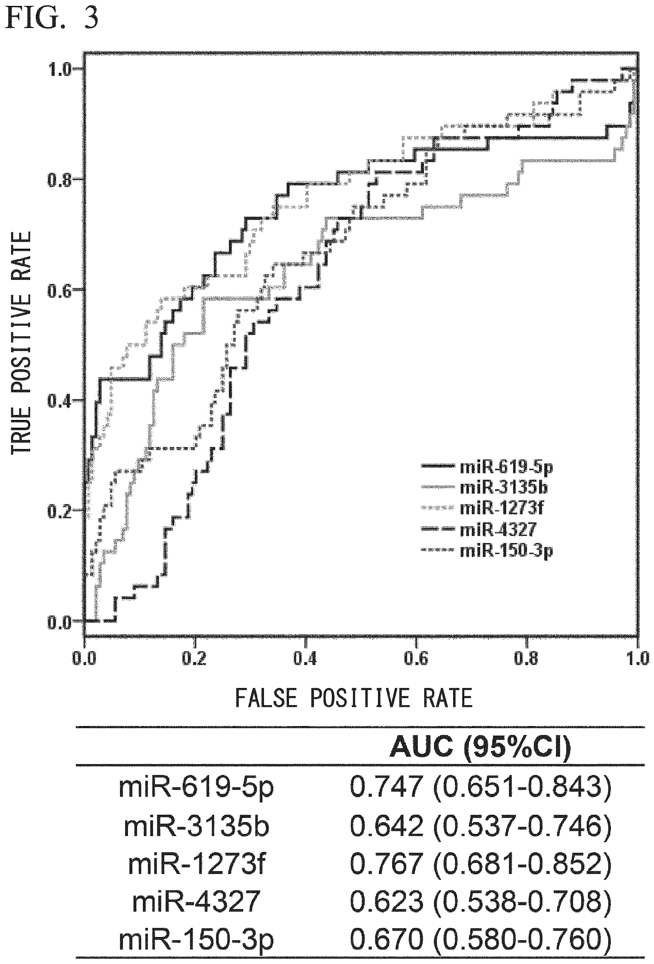

First, miRNA array analysis was performed using the (i) comprehensive analysis cohort of 9 cases, and a plurality of miRNAs that had been significantly elevated or decreased in the urine of esophageal cancer cases were identified. Next, expression analysis was performed with the (ii) biomarker establishment cohort. Specifically, for the (ii) biomarker establishment cohort of 192 cases, each miRNA extracted in (i) was measured by a qPCR method, and as a result, it was recognized in a univariate analysis that miR-619-5p, miR-3135b, miR-1273f, miR-4327, and miR-150-3p were significantly highly expressed in the urine of esophageal cancer cases. No increase in accuracy was recognized even though an analysis combining a multivariate analysis and each factor was performed, and it was considered that each of the five kinds of miRNAs could serve alone as a biomarker for esophageal cancer (Fig. 2). Furthermore, the AUC in the determination of esophageal cancer was 0.747 for miR-619-5p, 0.642 for miR-3135b, 0.767 for miR-1273f, 0.623 for miR-4327, and 0.670 for miR-150-3p, and they all enabled meaningful determination of esophageal cancer; however, among them, miR-619-5p in urine and miR-1273f in urine enabled determination with particularly high accuracy (Fig. 3). The sensitivity/specificity of esophageal cancer determination was 81.3%/54.2% for miR-619-5p in urine and 81.3%/52.1% for miR-1273f in urine. In this analysis, the cutoff value (2^{-ΔCT} value serving as reference) of each biomarker was set as follows.
miR-619-5p: 0.265
miR-3135b: 1.15
miR-1273f: 0.035
miR-4327: 0.32
miR-150-3p: 0.0062

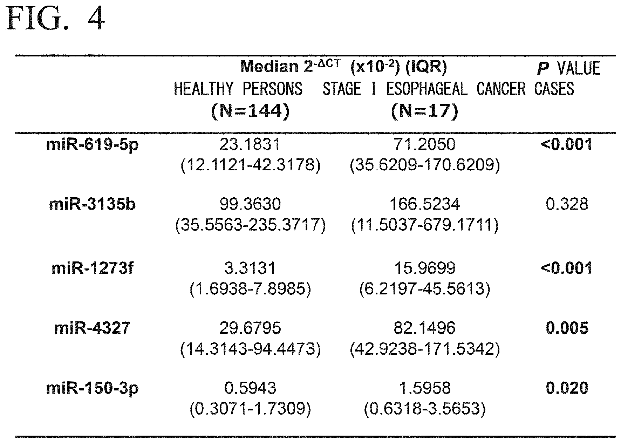

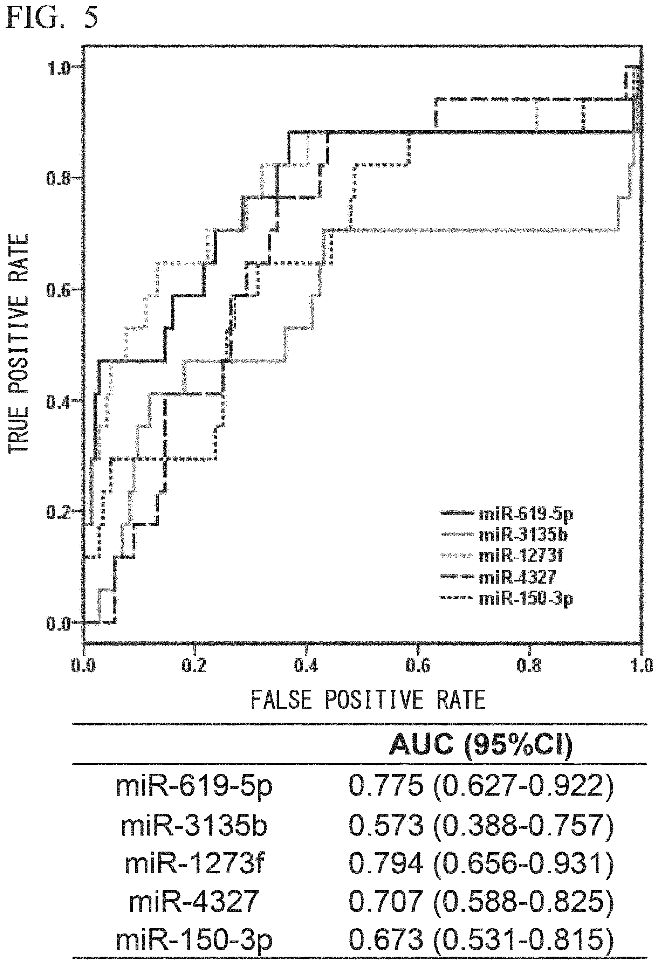

Next, even in the examination of early-stage esophageal cancer diagnostic ability in 144 cases of healthy persons and 17 cases of stage I esophageal cancer, regarding miR-619-5p, miR-1273f, miR-4327, and miR-150-3p, significantly high expression was recognized in the urine of the stage I esophageal cancer patients as compared with healthy persons (Fig. 4). The AUC in the determination of stage I esophageal cancer was 0.775 for miR-619-5p, 0.794 for miR-1273f, 0.707 for miR-4327, and 0.673 for miR-150-3p, and they all enabled meaningful determination of stage I esophageal cancer, while among them, miR-619-5p in urine and miR-1273f in urine enabled determination with particularly high accuracy (that is, highly accurate early diagnosis of esophageal cancer) (Fig. 5). The sensitivity/specificity of stage I esophageal cancer determination at the time of determining the cutoff value by an ROC analysis was 88.2%/63.2% for miR-619-5p in urine and 88.2%/59.7% for miR-1273f in urine. In this analysis, the cutoff value (2^{-ΔCT} value serving as reference) of each biomarker was set as follows.
miR-619-5p: 0.31
miR-1273f: 0.041
miR-4327: 0.35
miR-150-3p: 0.0062

### 3. Summary

From the above-described results, it was suggested that the expression levels of miR-619-5p, miR-1273f, miR-3135b, miR-4327, and miR-150-3p in urine (especially miR-619-5p and miR-1273f) are useful as novel, non-invasive esophageal cancer biomarkers.

### 4. Relationship with clinical stage

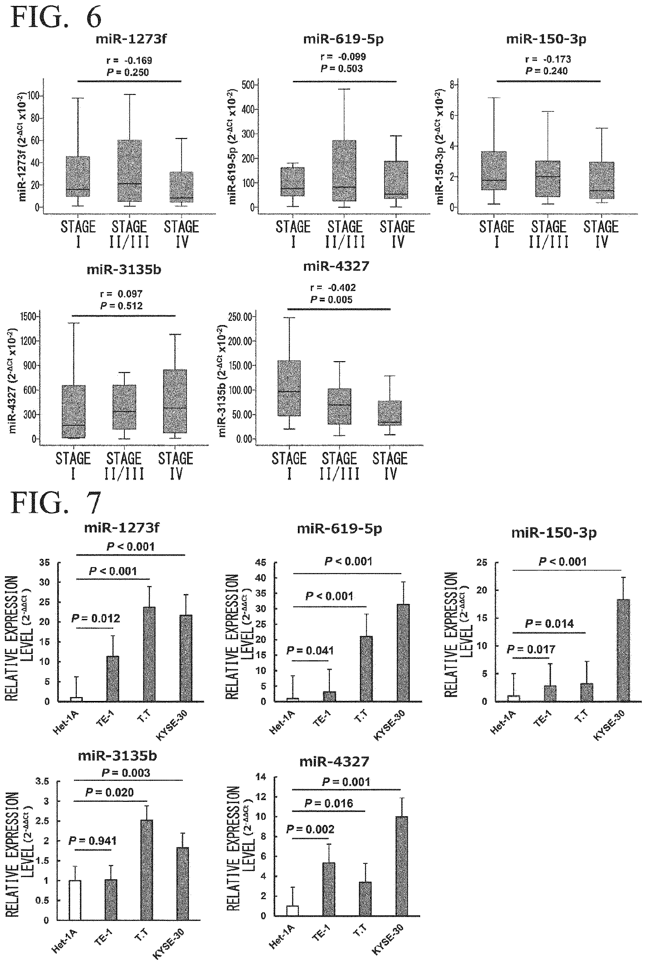

The relationship between the five kinds of miRNAs in urine, which are the identified biomarkers, and the clinical stage was investigated. miR-1273f, miR-619-5p, miR-150-3p, and miR-3135b showed no correlation between the miRNA expression in urine and the clinical stage (Fig. 6). That is, the expression of these miRNAs increased from stage I and subsequently remained high regardless of the progression of the disease stage (the plateau was reached at stage I). On the other hand, the miRNA expression in urine of miR-4327 was inversely correlated with the clinical stage (Fig. 6). That is, the miRNA expression was the highest in stage I, and the expression decreased as the disease stage progressed. The above-described results demonstrate that among the five kinds of identified miRNAs in urine, four kinds (miR-1273f in urine, miR-619-5p in urine, miR-150-3p in urine, and miR-4327 in urine) are biomarkers highly excellent in detection and identification of early-stage esophageal cancer. With regard to miR-3135b in urine, the median value of stage I esophageal cancer patients was about twice the median value of healthy persons, and a possibility that this may be utilized for the detection and identification of early-stage esophageal cancer was suggested.

### 5. Examination using cell lines

miRNAs were extracted from each of the culture fluids of normal human esophageal squamous cell lines (Het-1A) and three kinds of human esophageal squamous carcinoma cell lines (TE-1, T.T, KYSE-30), and expression analysis of five kinds of identified miRNAs was carried out. In the expression analysis, RNU6B was used as a standardization factor. The analysis results are shown in Fig. 7. All of the five kinds of miRNAs were significantly highly expressed in the culture fluids of human esophageal cancer cell lines as compared with normal cell lines. These results demonstrate that five kinds of miRNAs in urine (miR-1273f in urine, miR-619-5p in urine, miR-150-3p in urine, miR-3135b in urine, and miR-4327 in urine) are useful for the detection and identification of esophageal cancer.

### 6. Examination using tumor tissue preparation after excision

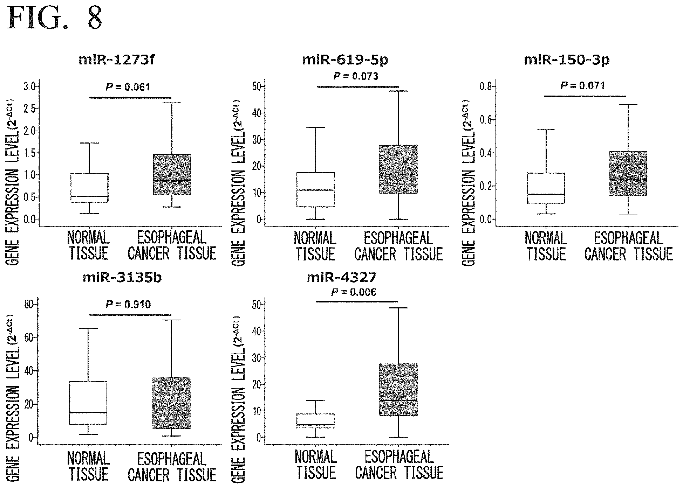

miRNAs were extracted from esophageal cancer tissue and surrounding normal tissue by utilizing formalin-fixed paraffin-embedded (FFPE) sections of 27 cases of esophageal cancer (16 cases of stage I early-stage esophageal cancer), in which tumor tissue preparations after excision were present, and the expression of five kinds of miRNA (miR-619-5p, miR-1273f, miR-3135b, miR-4327, miR-150-3p) was analyzed. In the expression analysis, RNU6B was used as a standardization factor. The analysis results are shown in Fig. 8. miR-4327 was significantly highly expressed in esophageal cancer tissue as compared with normal tissue, and miR-1273f, miR-619-5p, and miR-150-3p also showed a tendency of high expression. On the other hand, miR-3135b showed no significant difference between the two groups. This suggests a possibility that the number of cases may be small and the detection power may be insufficient (false negative), and a possibility that miR-3135b may be less useful in detecting early-stage cancer as compared with the other four kinds of miRNAs.

### [Industrial Applicability]

The present invention provides a biomarker useful for the diagnosis of esophageal cancer. The biomarker of the present invention can be detected in a urine specimen and enables a minimally invasive and convenient examination. Since urine is used as a specimen, it is possible to perform examinations and diagnoses non-invasively, easily, and at home which is extremely useful at, for example, the scene of primary screening. Furthermore, when the biomarker of the present invention is utilized, a highly accurate examination can be achieved.

The present invention can be carried out, for example, as one item of urine analysis in a health examination performed in a hospital, a health center, a medical examination vehicle, or the like. In addition, since urine is used as a specimen, an examination of new style, in which urine collected at home is sent to an examination facility and examined, can also be adopted.

The present invention is not limited to the description of the embodiments and examples of the above-described invention. Various modifications are also included in the present invention to the extent that those skilled in the art can easily conceive without departing from the description of the scope of claims. The contents of the articles, published patent gazettes, patent gazettes, and the like specified in the present specification are incorporated herein in their entirety by reference.

## Claims

1. An esophageal cancer biomarker, comprising:
at least one of microRNA selected from a group consisting of hsa-miR-619-5p in urine, hsa-miR-1273f in urine, hsa-miR-3135b in urine, hsa-miR-4327 in urine, and hsa-miR-150-3p in urine.

2. A method for examining esophageal cancer, comprising:
using an expression level of the esophageal cancer biomarker according to Claim 1 as an index.

3. The esophageal cancer biomarker according to Claim 1,
wherein the esophageal cancer biomarker is used for detecting early-stage esophageal cancer.

4. The esophageal cancer biomarker according to Claim 3,
wherein the esophageal cancer biomarker comprises hsa-miR-619-5p in urine, hsa-miR-1273f in urine, hsa-miR-4327 in urine, or hsa-miR-150-3p in urine.

5. A method for examining early-stage esophageal cancer, comprising:
using an expression level of the esophageal cancer biomarker according to Claim 3 or 4 as an index.

6. The method according to Claim 2, further comprising:
a step (1) which is a step of detecting the esophageal cancer biomarker in a specimen collected from an examinee and determining the expression level; and
a step (2) which is a step a step of determining esophageal cancer contraction based on the expression level determined in the step (1).

7. The method according to Claim 5, further comprising:
a step (1) which is a step of detecting the esophageal cancer biomarker in a specimen collected from an examinee and determining the expression level; and
a step (2) which is a step of determining early-stage esophageal cancer contraction based on the expression level determined in the step (1).

8. The method according to Claim 6 or 7,
wherein in the step (1), the expression level of the esophageal cancer biomarker is determined by conducting standardization using hsa-miR-4669 and hsa-miR-6756-5p as standardization factors.

9. The method according to any one of Claims 6 to 8,
wherein the esophageal cancer biomarker to be detected in step the (1) is hsa-miR-619-5p in urine or hsa-miR-1273f in urine.

10. The method according to any one of Claims 6 to 9,
wherein the esophageal cancer biomarker is detected by a qRT-PCR method.

11. A kit for esophageal cancer examination, comprising:
a reagent for detecting the esophageal cancer biomarker according to Claim 1; and
an instruction manual.

12. A kit for esophageal cancer examination, comprising:
a reagent for detecting the esophageal cancer biomarker according to Claim 3 or 4; and
an instruction manual.

13. The kit for esophageal cancer examination according to Claim 11 or 12,
wherein the reagent is at least one selected from a group consisting of a reagent for detecting hsa-miR-619-5p in urine, a reagent for detecting hsa-miR-1273f in urine, a reagent for detecting hsa-miR-3135b in urine, and a reagent for detecting hsa-miR-4327 in urine, and a reagent for detecting hsa-miR-150-3p in urine.

14. The kit for esophageal cancer detection according to any one of Claims 11 to 13, the kit further comprising at least one of elements selected from a group consisting of a reagent for microRNA extraction, a standardization factor-specific primer, a DNA polymerase, a reverse transcriptase, dNTP, a urine specimen collecting vessel, a reaction device, and a detection device.
